(19) Europäisches Patentamt
European Patent Office
Office européen des brevets

(11) **EP 2 566 427 B1**

(12) **EUROPÄISCHE PATENTSCHRIFT**

(45) Veröffentlichungstag und Bekanntmachung des Hinweises auf die Patenterteilung:
**06.06.2018 Patentblatt 2018/23**

(21) Anmeldenummer: **11715173.8**

(22) Anmeldetag: **12.04.2011**

(51) Int Cl.:
*A61F 13/539* (2006.01)     *B32B 5/02* (2006.01)
*B32B 5/04* (2006.01)       *B32B 5/26* (2006.01)
*B32B 7/12* (2006.01)       *A61F 13/49* (2006.01)
*A61F 13/53* (2006.01)      *A61F 13/537* (2006.01)
*A61F 13/15* (2006.01)

(86) Internationale Anmeldenummer:
**PCT/EP2011/001806**

(87) Internationale Veröffentlichungsnummer:
**WO 2011/137962 (10.11.2011 Gazette 2011/45)**

(54) **VERFAHREN ZUR HERSTELLUNG EINES ELASTISCHEN VLIESSTOFFVERBUNDMATERIALS**

METHOD FOR PRODUCING AN ELASTIC NONWOVEN COMPOSITE MATERIAL

PROCÉDÉ POUR LA FABRICATION D'UN MATÉRIAU COMPOSITE ÉLASTIQUE EN NON-TISSÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.05.2010 DE 102010019702**

(43) Veröffentlichungstag der Anmeldung:
**13.03.2013 Patentblatt 2013/11**

(73) Patentinhaber: **Paul Hartmann AG**
**89522 Heidenheim (DE)**

(72) Erfinder: **MALOWANIEC, Krzysztof, Daniel**
**89522 Heidenheim (DE)**

(56) Entgegenhaltungen:
WO-A2-02/24132        US-A- 5 219 633
US-A- 5 389 095       US-A- 6 049 915
US-A1- 2007 148 433   US-A1- 2010 022 151

EP 2 566 427 B1

**Beschreibung**

Die Erfindung betrifft ein Verfahren zur Herstellung eines elastischen Vliesstoffverbundmaterials zur Verwendung in einem Einwegartikel, wie in einem OP-Bekleidungsartikel oder Hygieneartikel, insbesondere in einem absorbierenden Inkontinenzartikel.

Absorbierende Einwegartikel wie Hygieneartikel oder OP-Bekleidungsartikel umfassen häufig elastische Komponenten, um die Passform des Artikels und damit den Tragekomfort zu verbessern.

Die elastischen Komponenten umfassen häufig eine Vielzahl von Elastifizierungsmitteln, häufig in Form von elastischen Fäden, die im vorgespannten Zustand mit im Wesentlichen unelastischen Chassismaterialien zumeist klebend verbunden werden. Dabei ist zum Beispiel in Windelhosen üblicherweise ein Hüftrandbereich vorzugsweise durchgehend in Umfangsrichtung elastifiziert. Auch im Vorderbereich und im Rückenbereich sind bei bekannten Windelhosen Elastifizierungsmittel vorgesehen. Desgleichen werden die Beinöffnungen umgebende bzw. die Beinöffnungen bildende Umfangsbereiche zumindest abschnittsweise elastisch ausgestaltet, damit dort ein weitgehend dichtendes Anliegen des Hygieneartikels an die Hautoberfläche des Benutzers gewährleistet ist, um das seitliche Austreten von Körperausscheidungen zu verhindern. Auch aufstehende Bündchenelemente, die neben den elastischen Beinöffnungen einen weiteren seitlichen Auslaufschutz bieten, werden bei bekannten Windelhosen bereits eingesetzt und sind (beispielsweise aus EP-1184017-A1, EP-1199058-A1, EP-1308148-A2) bekannt.

Es ist ferner bekannt, zwischen Hüftrand und Schrittbereich Vliesstoffverbundmaterialien zu verwenden mit im Wesentlichen in einer Querrichtung erstreckten Elastifizierungsmitteln in Form von elastischen Fäden, die im vorgespannten Zustand mit im Wesentlichen unelastischen Chassismaterialien zumeist klebend verbunden werden (stretch-bonding).

Ein Verfahren zur Herstellung eines derartigen Verbundmaterials offenbart bereits die WO/0188245. Das Verfahren umfasst das Extrudieren der elastischen Filamente und das Verbinden der Filamente mit einer Vliesstoffbahn mittels eines Klebstoffes, wobei die elastischen Filamente in vorgespanntem Zustand mit der Vliesstoffbahn verbunden werden (stretch-bonding).

Nachteil dieses Verfahrens ist die Notwendigkeit der Verwendung großer Klebermengen zur Verbindung der elastischen Filamente mit der Vliesstoffbahn.

Die EP2136753A2 offenbart bereits ein Verfahren zur Herstellung eines elastischen Vliesstoffverbundmaterials umfassend das Zuführen und Fördern einer ersten Vliesstofflage in einer Längsrichtung; Zuführen einer Vielzahl elastischer Filamente; Verbinden der Vielzahl elastischer Filamente mit einer ersten Seite der Vliesstofflage zur Bildung eines Materialverbundes; Zumindest bereichsweises Überdehnen der ersten Vliesstofflage in der Längsrichtung durch Hindurchführen des Materialverbundes durch den Spalt eines ersten Paars profilierter, ineinander greifender Dehnwalzen, wodurch der Materialverbund in der Längsrichtung gestreckt wird, wobei die elastischen Filamente derart angeordnet werden, dass sie im Wesentlichen in der Längsrichtung orientiert sind, so dass sie höchstens 10 Schnittpunkte miteinander pro cm$^2$ aufweisen.

Während im durch die WO01/88245 dokumentierten Stand der Technik die verfügbare Dehnung des Laminates durch das Stretch-Bonding, also das Verbinden der vorgespannten elastischen Filamente mit der Vliesstofflage gewährleistet wird, wird mit der EP2136753A2 zumindest ein wesentlicher Teil der verfügbaren Dehnung dadurch erzielt, dass die Vliesstofflage im bereits verbundenen Materialverbund einem nachträglichen Überdehnen, das heißt einer permanenten Verformung unterzogen wird, während sich die elastischen Filamente während des Streckvorganges im Wesentlichen elastisch verformen. Das Überdehnen erfolgt durch Hindurchführen des Materialverbundes durch den Spalt eines ersten Paars profilierter, ineinander greifender Dehnwalzen. Das Verbinden von elastischen Filamenten und der Vliesstofflage kann also zu einem Zeitpunkt stattfinden, zu dem die elastischen Filamente keiner signifikanten Vorspannung unterzogen sind. Dies hat den Vorteil, dass den Haltekräften zwischen der Vliesstofflage und den elastischen Filamenten im Zuge der Herstellung des Vliesstoffverbundmaterials weitaus weniger Kräfte entgegengesetzt werden und mithin weniger Klebstoff verwendet werden muss oder gar auf den Einsatz eines zusätzlichen Haftvermittlers ganz verzichtet werden kann.

Das in EP2136753A2 offenbarte Vliesstoffverbundmaterial ist jedoch lediglich als Hüll- oder Chassismaterial für Hygiene- oder OP-Bekleidungsartikel geeignet. Aufgabe der vorliegenden Erfindung war es daher, das Verbundmaterial für weitere Komponenten eines Hygiene- oder OP-Bekleidungsartikel geeignet zu gestalten und hierfür ein vorteilhaftes Herstellverfahren bereit zu stellen.

Gelöst wird diese Aufgabe durch ein Verfahren mit den Merkmalen des Anspruch 1. Indem eine Vielzahl partikelförmiger Materialien, wie insbesondere superabsorbierende Materialien, oder geruchsadsorbierende Materialien, wie zum Beispiel Zeolithe oder Cyclodextrine, sandwichartig zwischen die Vliesstofflagen, und zwar in den durch die ungebundenen Bereiche gebildeten Freiraum eingebracht werden, kann der Vliesstoffverbund als Flüssigkeiten absorbierende oder Gerüche adsorbierenden Komponente eines Hygiene- oder OP-Bekleidungsartikels Verwendung finden. Durch das thermische Verbinden mittels Schmelzbindungen, kann zwar nicht ausgeschlossen werden, dass vereinzelt partikelförmiges Material von den Schmelzbindungen mit erfasst wird und damit zumindest anteilig deaktiviert wird; eine Vielzahl, insbesondere mindestens 60 Gew.-%, vorzugsweise mindestens 75 Gew.-% und weiter vorzugsweise mindestens 85 Gew.-% des partikelförmigen Materials verbleibt aber im Freiraum. Die Beschränkung der Partikelgrößenspannweite von mindestens 80-Gew.-% der Partikel auf einen Bereich zwischen 45 μm und 1200 μm stellt sicher, dass die Partikel bei der bestimmungsgemäßen Verwendung des Vliesstoffverbundes einerseits keine unangenehmen Hartstellen bilden und andererseits die Gefahr des unerwünschten Verlustes von Partikeln durch die offenporigen Vliesstofflagen reduziert ist. Vorzugsweise weisen mindestens 90 Gew.-%, insbesondere mindestens 95 Gew.-% der partikelförmigen Materialien eine Partikelgröße von 45-1200 μm, insbesondere von 45-850 μm, weiter insbesondere von 45-600 μm auf.

Die Partikelgröße wird im Rahmen dieser Erfindung gemäß der Standard-Testmethode WSP 220.2 (05) gemessen. Die Testmethode WSP 220.2 (05) ist in "Standard Test Methods for the Nonwovens and Related Industries", Ausgabe 2008 beschrieben (herausgegeben von EDANA und INDA). WSP 220.2 (05) nach EDANA/INDA ist eine Standard-Testmethode zur Ermittlung der Partikelgrößen von superabsorbierenden Polymermaterialien. Im Zusammenhang mit der vorliegenden Erfindung wird die Bestimmung der Partikelgrößen von anderen Partikeln, wie zum Beispiel von geruchsadsorbierenden Partikeln, auf entsprechende Weise durchgeführt. Verwendet wird jeweils ein Retsch VE 1000 sieve shaker. In Ergänzung zu den nach WSP 220.2 (05) vorgesehenen Standardsieben wird ein Sieb mit einer Porengröße von 1200 μm verwendet. Vor der Siebung werden die Partikel bei 23±2 °C und 50±5 % Luftfeuchte über 24 h klimatisiert.

Vorzugsweise stellt der Freiraum ein im Vergleich zum Gesamtvolumen der partikelförmigen Materialien relativ großes Volumen zur Verfügung. Vorzugsweise ist das Geamtvolumen des Freiraums zumindest um den Faktor 2, insbesondere zumindest um den Faktor 3 und weiter insbesondere zumindest um den Faktor 5 größer als das Volumen der Summe der in den Freiraum eingebrachten partikelförmigen Materialien. Dies reduziert weiter die Gefahr der Bildung von Hartstellen und ermöglicht außerdem im Falle von superabsorbierenden partikelförmigen Materialien das weitgehend ungehinderte Quellen der Materialien bei bestimmungsgemäßer Absorption von Körperflüssigkeit.

In bevorzugter Ausführungsform umfassen die partikelförmigen Materialien superabsorbierende Materialien oder bestehen daraus. Der Anteil der superabsorbierenden Partikel am Gesamtgewicht des Vliesstoffverbundes beträgt vorzugsweise 10-200 Gew.-%, insbesondere 20-150 Gew.-%, weiter insbesondere 30-120 Gew.-%.

Im Rahmen dieser Erfindung wird unter dem Ausdruck "superabsorbierendes Material" ein superabsorbierendes Polymer insbesondere ein Polymer verstanden, welches gemäß der Standard-Testmethode WSP 240.2 (05) einen w-Wert ("free swell capacity") von mindestens 10 g/g, vorzugsweise mindestens 20 g/g, weiter vorzugsweise höchstens 80 g/g, weiter vorzugsweise höchstens 70 g/g aufweist. Die Testmethode WSP 240.2 (05) zur Bestimmung des w-Wertes ist in "Standard Test Methods for the Nonwovens and Related Industries", Ausgabe 2008 beschrieben (herausgegeben EDANA und INDA). WSP 240.2 (05) nach EDANA/INDA ist eine Standard-Testmethode zur Ermittlung des w-Wertes ("free swell capacity") von superabsorbierendem Polyacrylat-Pulver. Nach WSP 240.2 (05) wird die freie Aufnahmekapazität für eine 0,9 % (Gewichtsprozent) Kochsalzlösung bestimmt. Im Zusammenhang mit der vorliegenden Erfindung wird die Bestimmung des w-Wertes von superabsorbierendem Material, bei dem es sich nicht um Polyacrylat-Pulver handelt, auf entsprechende Weise durchgeführt.

Das die Vliesstofflagen verbindende Bindemuster ist vorzugsweise ein Punktbindemuster, das heißt die Vliesstofflagen sind durch punktuell, thermisch erzeugte Schweißbindungen verbunden, so dass punktartige gebundene Bereiche von ungebundenen, Freiräume bildenden Bereichen umgeben sind. Vorteilhafterweise ist das Bindemuster derart ausgeführt, dass jedes der elastischen Filamente durch mindestens einen Bindebereich, insbesondere mindestens zwei, weiter insbesondere mindestens drei Bindebereiche verläuft.

Denkbar und vorteilhaft ist auch, das Bindemuster derart zu gestalten, dass inselartig ungebundene, die Freiräume bildende Bereiche von gebundenen Bereichen umgeben sind. Diesbezüglich wird beispielhaft auf die in EP1806989A1 offenbarten Bindemuster verwiesen. Solchenfalls wären die partikelförmigen Materialien innerhalb des Vliesstoffverbundes sicherer fixiert.

Denkbar wäre auch, die Vliesstofflagen und die elastischen Filamente zusätzlich zum thermischen Verbinden durch einen Haftvermittler, insbesondere durch geringe Mengen eines diskontinuierlich, also nicht vollflächig, beispielsweise raster- oder punktförmig aufgetragenen Hotmeltklebstoffes zu verbinden. Dieser würde vorzugsweise über an sich bekannte Module wie Hotmeltsprüh- oder schlitzkopf- oder Druckauftragsvorrichtungen auf eine der oder beide Vliesstofflagen aufgetragen werden. Auch dies trägt dazu bei, die partikelförmigen Materialien sicherer innerhalb des Vliesstoffverbundes zu fixieren.

In einer bevorzugten Weiterbildung der Erfindung werden die elastischen Filamente im Wesentlichen ungedehnt, insbesondere mit einer Vorspannung von weniger als 1,5, insbesondere weniger als 1,3, weiter insbesondere weniger als 1,2, weiter insbesondere weniger als 1,1 und vorzugsweise weniger als 1,05 mit der ersten und/oder zweiten Vliesstofflage verbunden. Hierbei bedeutet eine Vorspannung von 1,5, das Dehnen der elastischen Filamente um 50 %, also beispielsweise, das Dehnen eines 10 cm langen Filamentabschnittes auf 15 cm.

In einer bevorzugten Form des Verfahrens wird der Materialverbund beim Hindurchführen des Materialverbundes durch den Spalt eines ersten Paars profilierter, ineinander greifender Dehnwalzen in Längsrichtung um 35-300%, insbesondere um 50-200% gestreckt. Unter Dehnung oder Streckung wird hierbei das Verhältnis der Längenänderung zur Ausgangslänge verstanden. Ein Abschnitt einer Ausgangslänge von beispielsweise 100 cm, welcher auf eine Länge von 150 cm gedehnt wird, würde nach diesem Verständnis um 50% gedehnt werden.

Je nach Ausmaß der mit der Streckung verbundenen permanenten Dehnung der Vliesstofflage resultiert daraus ein Vliesstoffverbundmaterial mit einer in Längsrichtung verfügbaren Dehnung (Dehnbarkeit) von vorzugsweise 35-300%, insbesondere 50-200%.

Unter verfügbarer Dehnung oder Dehnbarkeit wird das Verhältnis der möglichen Längenänderung zur Ausgangslänge eines Abschnittes des Vliesstoffverbundmaterials verstanden. Die mögliche Längenänderung wird hierbei nach der in EP 2136753A2 beschriebenen Methode ermittelt.

Nach einem weiteren Erfindungsgedanken kann der Materialverbund nach dem Hindurchführen des Materialverbundes durch den Spalt eines ersten Paars profilierter, ineinander greifender Dehnwalzen durch einen jeweiligen Spalt weiterer Paare, insbesondere zweier weiterer Paare, vorzugsweise eines weiteren Paares profilierter, ineinander greifender Dehnwalzen hindurchgeführt werden. Solchenfalls kann die Überdehnung stufenweise erfolgen. In Weiterbildung dieses Erfindungsgedankens wird der Materialverbund durch das Hindurchführen des Materialverbundes durch den Spalt des ersten Paars profilierter, ineinander greifender Dehnwalzen vorzugsweise stärker gedehnt als durch das Hindurchführen des Materialverbundes durch den jeweiligen Spalt jedes weiteren Paares profilierter, ineinander greifender Dehnwalzen.

Aus wirtschaftlichen Gründen ist es vorteilhaft, dass die Vliesstofflage oder die Vliesstofflagen aus unelastischem Material gebildet sind.

In einer ersten Alternative des erfindungsgemäßen Verfahrens ist vorgesehen, die elastischen Filamente in an sich bekannter Weise in einem separaten Herstellprozess, also offline zu fertigen, anschließend endlos auf Rollen zu wickeln und die elastischen Filamente im Zuge der Herstellung des Vliesstoffverbundmaterials von der Rolle abzuwickeln. Denkbar und besonders vorteilhaft ist hingegen, dass das Verfahren der Herstellung des Vliesstoffverbundmaterials das Extrudieren der elastischen Filamente mit umfasst. Solchenfalls würde man von einer inline-Fertigung der elastischen Filamente sprechen.

Wie bereits weiter oben angedeutet erweist es sich insbesondere aus Kostengründen als vorteilhaft, die elastischen Filamente direkt, also insbesondere ohne weiteren Haftvermittler mit der Vliesstofflage zu verbinden. Hierzu können die elastischen Filamente ein Polymer mit permanenter Haftkraft enthalten. Im Falle der inline-Fertigung der elastischen Filamente kann die dauerhafte Verbindung zwischen elastischen Filamenten und Vliesstofflage auch dadurch erfolgen, dass die elastischen Filamente kurz nach der Extrusion in noch nicht völlig erstarrtem Zustand mit der Vliesstofflage in Kontakt gebracht werden. Denkbar und vorteilhaft ist auch, die elastischen Filamente gemeinsam mit den Vliesstofflagen durch einen Pressspalt, insbesondere einen beheizbaren Pressspalt eines Walzenpaars, insbesondere eines Kalanderwalzenpaares hindurchzuführen. Hierbei werden die Vliesstofflagen und die elastischen Filamente durch ein Bindemuster, also ein Muster gebundener und nicht gebundener Bereiche, insbesondere ein Punktbindemuster thermisch, also durch thermisch erzeugte Schweißbindungen verbunden werden. Vorteilhafterweise ist das Bindemuster derart ausgeführt, dass jedes der elastischen Filamente durch mindestens einen Bindebereich, insbesondere mindestens zwei, weiter insbesondere mindestens drei Bindebereiche verläuft.

Im Zusammenhang mit dem Schritt des Streckens des Vliesstoffverbundmaterials in seiner Längsrichtung kann es sich als vorteilhaft erweisen, nach dem Überdehnen durch Hindurchführen des Materialverbundes durch den Spalt eines Paares profilierter, ineinander greifender Dehnwalzen, die Bahn des Vliesstoffverbundmaterials in Querrichtung zu fixieren, um einer Einschnürung der Bahn in Querrichtung, das heißt einer Verringerung der Erstreckung in der Querrichtung entgegenzuwirken. Gleichwohl kann es vorteilhaft sein, gezielt ein geringes Maß an Einschnürung in Querrichtung, insbesondere weniger als 50%, weiter insbesondere weniger als 30%, weiter insbesondere weniger als 20% und ganz besonderes weniger als 10% zuzulassen. Die Einschnürung in Querrichtung in % wird hierbei nach folgender Formel berechnet:

$$Einschnürung[\%] = \frac{LQ0 - LQ1}{LQ0} x100\%,$$

wobei LQ0 die Länge der Bahn in Querrichtung vor dem Verfahrensschritt des Überdehnens ist und LQ1 die Länge der Bahn in Querrichtung nach dem Verfahrensschritt des Überdehnens ist.

Solchenfalls ist es möglich, das Vliesstoffverbundmaterial mit einer zumindest geringfügigen Dehnbarkeit auch in Querrichtung auszustatten.

Die erste und/oder die zweite Vliesstofflage können in an sich bekannter Weise in einem separaten Herstellprozess, also offline nach einem der im Stand der Technik bekannten Verfahren, wie zum Beispiel Spinnvlies-, Kardenvlies-, Spunlacevlies- oder Melblownvliesherstellverfahren gefertigt und anschließend endlos auf Rollen gewickelt werden, um die vorgefertigten Vliesstofflagen dann im Zuge der Herstellung des elastischen Vliesstoffverbundmaterials von der Rolle abzuwickeln. Denkbar und vorteilhaft ist alternativ, dass das Verfahren der Herstellung des elastischen Vliesstoffverbundmaterials die inline-Fertigung mindestens einer, vorzugsweise beider Vliesstofflagen, insbesondere nach dem Spinnvliesherstellverfahren umfasst.

Die erste Vliesstofflage und vorzugsweise auch die zweite Vliesstofflage weisen vorzugsweise ein Flächengewicht von 5-30 g/m$^2$, insbesondere von 8-25 g/m$^2$, weiter insbesondere von 10-22 g/m$^2$, ganz besonders von 12-18 g/m$^2$ auf.

Die erste und/oder die zweite Vliesstofflage sind insbesondere in Längsrichtung und vorzugsweise auch in Querrichtung im Wesentlichen unelastisch. Dies trägt dazu bei, Kosten zu sparen und die weiter unten beschriebene zweiphasige Dehnungscharakteristik möglich machen zu können. Unter einer unelastischen Vliesstofflage wird im Rahmen der vorliegenden Erfindung ein Vliesstoffmaterial verstanden, das nach einer einmaligen Dehnung eines 25 mm breiten Materialstreifens um 30 % seiner Ausgangslänge bei einer Dehngeschwindigkeit von 500 mm/min entweder reißt oder bei anschließender sofortiger Entlastung eine permanente Dehnung von mindestens 7,5 % aufweist. Dies bedeutet, dass beispielsweise ein 100 mm langer Materialstreifen, der auf eine Länge von 130 mm gedehnt wurde, nach Entlastung eine Länge von mindestens 107,5 cm aufweist.

In Weiterbildung der Erfindung wird ferner vorgeschlagen, das Strecken des Vliesstoffmaterials derart auszuführen, dass erste Bereiche der Vliesstofflage stärker überdehnt werden als zweite Bereiche der Vliesstofflage. Insbesondere vorteilhaft ist, die zweiten Bereiche im Wesentlichen gar nicht zu überdehnen. Vorzugsweise sind die ersten und zweiten Bereiche dann alternierend, streifenförmig quer zu der Längsrichtung der Bahn des Vliesstoffverbundmaterials angeordnet. Dies hat zur Folge, dass die Dehnungscharakteristik des Vliesstoffverbundmaterials in Gebrauch durch zwei Phasen gekennzeichnet ist. Die ersten stärker überdehnten Bereiche setzen dem Dehnen in Längsrichtung zunächst einen ersten, geringen Widerstand entgegen. Nach maximaler Ausdehnung der stark überdehnten Bereiche erfordert das weitere Dehnen der weniger stark oder gar nicht überdehnten Bereiche dann eine höhere Kraft, welche deutlich über den Kräften liegt, die bei normalem Gebrauch zu erwarten sind. Diese Zweiphasigkeit gibt dem Anwender einen Hinweis auf die maximale Dehnbarkeit des Materials, noch lange bevor das Material der Gefahr des Zerreißens ausgesetzt wird.

In einer weiteren vorteilhaften Ausführung der Erfindung beträgt die Stärke der elastischen Filamente 4-700 dtex, insbesondere 50-300 dtex und weiter insbesondere 100-200 dtex. In Weiterbildung dieses Erfindungsgedankens enthalten die erste und vorzugsweise auch die zweite Vliesstofflage Fasern mit einem ersten Titer, gemessen in dtex, oder bestehen daraus, während die elastischen Filamente einen zweiten Titer aufweisen, wobei der zweite Titer größer ist, vorzugsweise um bis zu 100% größer, besonders bevorzugt um bis zu 200% größer, insbesondere um bis zu 500% größer, vorzugsweise jedoch um höchstens 5000%, besonders bevorzugt um höchstens 4000 % und ganz besonders bevorzugt um höchstens 3000% größer ist als der erste Titer. Der Anteil des Gewichts der elastischen Filamente am Gesamtgewicht des Vliesstoffverbundmaterials beträgt dabei vorzugsweise weniger als 40 %, insbesondere weniger als 30 %, weiter insbesondere weniger als 20 %, weiter insbesondere weniger als 15 %, vorzugsweise aber mehr als 3%.

Die elastischen Filamente enthalten oder bestehen vorzugsweise aus einem thermoplastischen Polymer insbesondere einem extrudierbaren Polymer ausgewählt aus der Gruppe Polyurethane, elastische Polyester, elastische Polyamide, elastische Polyolefine oder elastische Blockcopolymere. Bevorzugte extrudierbare elastische Polymere offenbaren die WO01/88245 und die WO06/124092, die diesbezüglich vollumfänglich zum Offenbarungsgehalt der vorliegenden Erfindung gemacht werden.

Weiter bevorzugt enthalten die elastischen Filamente eine Polymerkomponente mit permanenter Haftkraft oder die elastischen Filamente bestehen aus einer Polymerkomponente mit permanenter Haftkraft.

Der Abstand der elastischen Filamente voneinander in Querrichtung beträgt vorzugsweise 0,5-10 mm, besonders bevorzugt 1-5 mm, insbesondere 1,2 - 3,5 mm.

Die Wasserdampfdurchlässigkeit des Vliesstoffverbundmaterials beträgt insbesondere wenigstens 300g/m$^2$/24h , weiter insbesondere wenigstens 1000g/m$^2$/24h, weiter insbesondere wenigstens 2000g/m$^2$/24h, weiter insbesondere wenigstens 3000g/m$^2$/24h, weiter insbesondere wenigstens 4000g/m$^2$/24h, weiter insbesondere höchstens 6000g/m$^2$/24h gemessen nach DIN 53 122-1 (Ausgabe: 2001-08).

Die vorliegende Erfindung betrifft außerdem ein Vliesstoffverbundmaterial herstellbar oder hergestellt nach dem Verfahren wie zuvor gekennzeichnet. Das Vliesstoffverbundmaterial mit einer Längsrichtung und einer senkrecht dazu verlaufenden Querrichtung umfasst eine zumindest bereichsweise überdehnte erste und zweite Vliesstofflage mit je einer ersten Seite und einer zweiten Seite, sowie eine Vielzahl im Wesentlichen in der Längsrichtung orientierter elastischer Filamente, die zumindest mit der ersten Seite der Vliesstofflage verbunden sind, wobei die elastischen Filamente derart angeordnet sind, dass sie höchstens 10 Schnittpunkte miteinander pro cm$^2$ aufweisen, wobei das Vliesstoffverbundmaterial in der Längsrichtung vorzugsweise eine Dehnbarkeit von 35-300% aufweist und wobei partikelförmige Materialien sandwichartig zwischen die erste und zweite Vliesstofflage eingebracht sind, wobei mindestens 80 Gew.-% der partikelförmigen Materialien eine Partikelgröße von 45-1200 $\mu$m aufweisen, und wobei die Vliesstofflagen zu einem Materialverbund mittels eines Bindemusters, welches gebundene Bereiche und ungebundene Bereichen umfasst, verbunden sind, wobei durch das Bindemuster erste Schmelzbindungen direkt zwischen erster und zweiter Vliesstofflage erzeugt sind, und wobei die ungebundenen Bereiche zwischen der ersten und der zweiten Vliesstofflage einen Freiraum bilden und wobei eine Vielzahl der partikelförmigen Materialien innerhalb dieses Freiraums angeordnet ist. Insbesondere sind die elastischen Filamente derart konsequent in der Längsrichtung ausgerichtet, dass sie höchstens 8, insbesondere höchstens 5, insbesondere höchstens 3, weiter insbesondere höchstens 1 und ganz besonders gar keinen, also 0 Schnittpunkte pro cm$^2$ Vliesstoffverbundmaterial miteinander aufweisen. Vorzugsweise verlaufen die elastischen Filamente in der Längsrichtung parallel zu einander.

Außerdem ist ein wegwerfbarer absorbierender Hygiene- oder OP-Bekleidungsartikel wie eine Inkontinenzwindel des offenen, mit Verschlusselementen versehenen Typs oder eine pantförmige Inkontinenzwindel oder eine Inkontinenzvorlage oder ein OP-Mantel mit mindestens einem Vliesstoffverbundmaterial herstellbar wie oben beschrieben Gegenstand der vorliegenden Erfindung.

Nach einem weiteren Erfindungsgedanken weist ein absorbierender Hygiene- oder OP-Bekleidungsartikel wie eine Inkontinenzwindel des offenen, mit Verschlusselementen versehenen Typs oder eine pantförmige Inkontinenzwindel oder eine Inkontinenzvorlage oder ein OP-Mantel mindestens ein Vliesstoffverbundmaterial wie oben beschrieben als eine Flüssigkeiten oder Gerüche ab- oder adsorbierende Komponente auf. Es ist denkbar und vorteilhaft, wenn diese Komponente gleichzeitig die hüllmaterial- und chassisbildende Komponente des Artikels bildet. In einer alternativen Ausführungsform weist der Artikel jedoch des Weiteren ein zweites Vliesstoffverbundmaterial, vorzugsweise wie in EP 2136753A2 beschrieben, als Chassis- oder Hüllmaterial auf. Solchenfalls kommt in einer mehrlagigen Anordnung innerhalb des Artikels das erfindungsgemäße Vliesstoffverbundmaterial relativ zum zweiten Vliesstoffverbundmaterial vorzugsweise körpernah zu liegen.

In einer bevorzugten Ausführungsform ist der Artikel eine Inkontinenzwindel in Pantform mit einem eine Hüftöffnung bildenden in Umfangsrichtung durchgehend geschlossenen Hüftrand und mit Beinöffnungen, und mit Längsseitenrandabschnitte aufweisendem Vorderteil und Rückenteil, wobei Vorderteil und Rückenteil voneinander in Längsrichtung des Hygieneartikels beabstandet sind, und mit einem das Vorderteil und Rückenteil in Längsrichtung des Hygieneartikels verbindenden Absorptionsteil, wobei der Absorptionsteils ein erfindungsgemäßes Vliesstoffverbundmaterial aufweist oder daraus besteht, und wobei der in Umfangsrichtung durchgehend geschlossene Hüftrand und die Beinöffnungen gebildet sind, indem Längsseitenrandabschnitte eines Vorderteils und eines Rückenteils herstellerseitig miteinander verbunden sind, und wobei das Vorderteil und/oder das Rückenteil ein zweites Vliesstoffverbundmaterial, vorzugsweise wie in EP 2136753A2 beschrieben, aufweisen oder daraus bestehen und wobei die Längsrichtung des zweiten Vliesstoffverbundmaterials im Wesentlichen senkrecht zu einer Längsrichtung des Hygieneartikels verläuft..

In Weiterbildung dieses Erfindungsgedankens beträgt die Dehnbarkeit des Vorder- und/oder Rückenteil bildenden zweiten Vliesstoffverbundmaterials senkrecht zur Längsrichtung des Hygieneartikels 80-250 %, insbesondere 100-200 %. Die Dehnbarkeit des Absorptionsteils beträgt senkrecht zur Längsrichtung des Hygieneartikels vorzugsweise 90-260 %, insbesondere 110-210 %. Vorzugsweise ist die Dehnbarkeit des Absorptionsteils senkrecht zur Längsrichtung des Hygieneartikels größer als die Dehnbarkeit des zweiten Vliesstoffverbundmaterials. Dies hat den Vorteil, dass beim Anlegen des Hygieneartikels an den Benutzer Vorder- oder Rückenteil bis zu ihrer maximalen Dehnbarkeit verlängert werden können, ohne dass der Absorptionsteil Gefahr liefe durch diese Dehnung geschädigt zu werden.

Insbesondere beträgt die Erstreckung des Vliesstoffverbundmaterials des Vorderteils und/oder des Rückenteils in Längsrichtung des Hygieneartikels 8-50 cm, insbesondere 10-40 cm und weiter insbesondere 12-30 cm.

Weitere Merkmale, Einzelheiten und Vorteile der Erfindung ergeben sich aus den beigefügten Patentansprüchen und aus der zeichnerischen Darstellung und nachfolgenden Beschreibung bevorzugter Ausführungsformen der Erfindung. In der Zeichnung zeigt:

Figur 1    in schematisierter Form eine Seitenansicht einer Vorrichtung für den Betrieb eines erfindungsgemäßen Verfahrens

Figur 2    eine Schnittansicht entlang einer Linie A-A der Figur 1

Figur 3    eine Teilansicht eines Querschnittes der Figur 5 entlang der Linie B-B

Figur 4    eine vergrößerte Darstellung des Eingriffsbereich eines Dehnwalzenpaares

Figur 5    einen Teilabschnitt eines Materialverbundes in der Aufsicht

Figur 6    einen Querschnitt eines Teilabschnittes eines Materialverbundes entlang einer Linie B-B aus Figur 5

Figur 7    einen Teilabschnitt eines elastischen Vliesstoffverbundmaterials in der Aufsicht im entspannten Zustand

Figur 8    einen Teilabschnitt eines elastischen Vliesstoffverbundmaterials in gedehntem Zustand

Figur 9    in schematisierter Form eine Seitenansicht einer weiteren Vorrichtung für den Betrieb eines weiteren erfindungsgemäßen Verfahrens

Figur 10    eine Draufsicht auf eine erste Ausführungsform eines pantförmigen Hygieneartikels im flachgelegten Zustand vor der herstellerseitigen Verbindung von Längsseitenrandbereichen

Figur 11    eine perspektivische Ansicht eines pantförmigen Hygieneartikels nach der herstellerseitigen Verbindung von Längsseitenrandbereichen

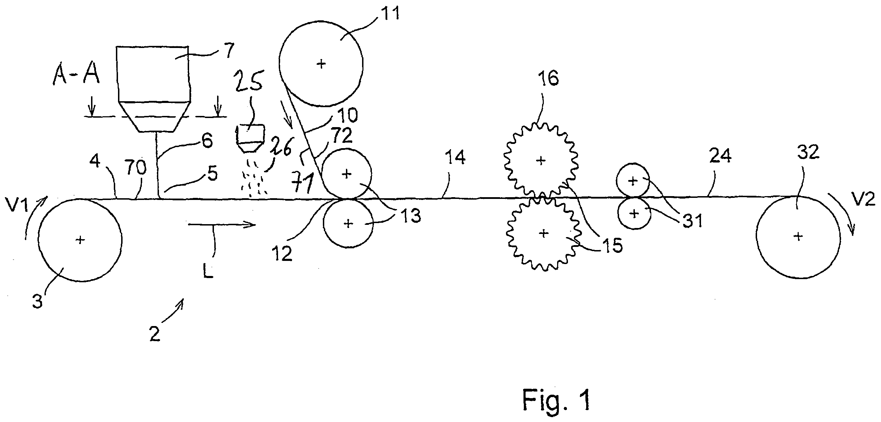

Figur 1 zeigt schematisch eine insgesamt mit dem Bezugszeichen 2 bezeichnete Vorrichtung zum Betreiben eines erfindungsgemäßen Verfahrens zur Herstellung eines elastischen Vliesstoffverbundmaterials. Von einer Rolle 3 wird eine erste bereits vorgefertigte unelastische Vliesstofflage 4, insbesondere eine PP-Spinnvlieslage mit einem Flächengewicht von insbesondere 18 g/m$^2$ mit einer Geschwindigkeit v1 abgerollt und in Längsrichtung L einem Kontaktbereich 5 zugeführt, in dem die elastischen Filamente 6 auf eine erste Seite 70 der ersten Vliesstofflage 4 im Wesentlichen spannungsfrei abgelegt werden. Die elastischen Filamente 6 werden im dargestellten Fall inline durch einen Extruder 7 erzeugt. Denkbar wäre natürlich auch, die elastischen Filamente 6 offline zu fertigen und dem Vliesstoffverbundmaterialherstellungsprozess als vorgefertigtes Material in an sich bekannter Weise zum Beispiel von der Rolle endlos zuzuführen.

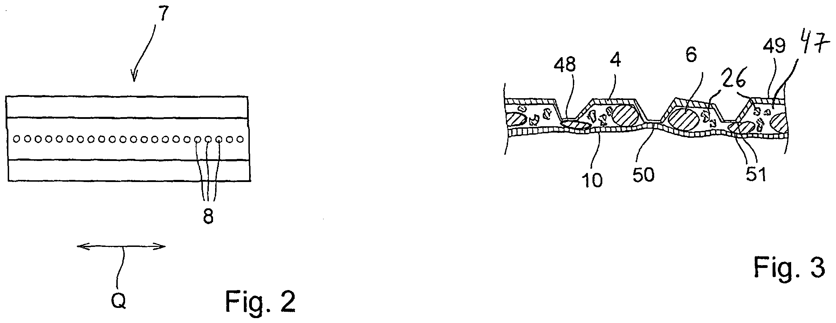

Figur 2 zeigt schematisch, nicht maßstabsgerecht, den Kopf des Extruders 7 im Querschnitt entlang einer Linie A-A. Erkennbar sind die Austrittsdüsen 8 für die elastischen Filamente 6. Der Abstand der Austrittsdüsen beträgt etwa 3 mm.

Der Kontakt der elastischen Filamente 6 mit der Vliesstofflage 4 im Kontaktbereich 5 erfolgt vorzugsweise zu einem Zeitpunkt, zu dem die extrudierten elastischen Filamente 6 noch nicht völlig erhärtet sind und somit in noch angeschmolzenem, mithin noch klebrigem Zustand.

Vor der Zuführung der zweiten Vliesstofflage 10 wird mittels eines schematisch angedeuteten Partikeldosierers 25 partikelförmiges Material 26, im dargestellten Fall superabsorbierendes Material auf die erste Seite 70 der ersten Vliesstofflage 4 aufgebracht.

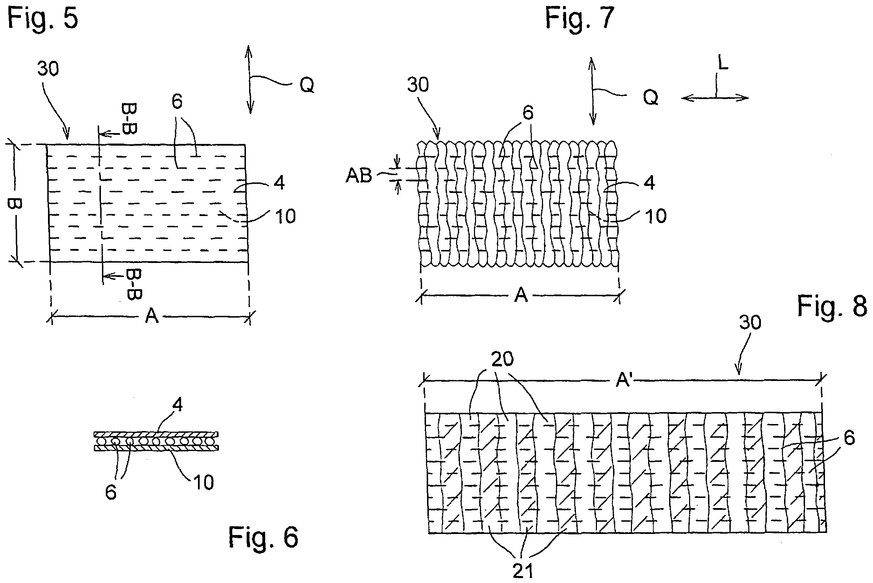

Das aus der ersten Vliesstofflage 4, den elastischen Filamenten 6 und dem partikelförmigen Material 26 bestehenden Vorlaminat 9 wird im dargestellten Fall beschichtet mit einer weiteren, zweiten unelastischen Vliesstofflage 10 mit einer ersten Seite 72 und einer zweiten Seite 71, insbesondere eine PP-Spinnvliesstofflage mit einem Flächengewicht von insbesondere 18 g/m$^2$, welche von einer Rolle 11 abgerollt wird. Die zweite Seite 71 der zweiten Vliesstofflage 10 ist dabei der ersten Seite 70 der ersten Vliesstofflage 4 zugewandt. Diese Lagenanordnung wird nachfolgend einem aus einem Kalanderwalzenpaar 13 gebildeten Pressspalt 12 zugeführt.. Das Kalanderwalzenpaar 13 umfasst eine erste glatte, sowie eine mit inselförmigen Erhebungen ausgeführte zweite, insbesondere beheizte Walze, so dass durch das Hindurchführen durch den Pressspalt Vorlaminat 9 und zweite Vliesstofflage 10 mittels Punktbindemuster, also der Bildung von punktartigen gebundenen Bereichen 48 und diese umgebenden ungebundenen Bereichen 49, dauerhaft verbunden werden können. Hierbei werden sowohl erste Schmelzbindungen 50 direkt zwischen erster und zweiter Vliesstofflage 4, 10 als auch zweite Schmelzbindungen 51 zwischen erster und/oder zweiter Vliesstofflage 4, 10 und den elastischen Filamenten 6 erzeugt. Dabei sind die Vliesstofflagen 4, 10 so angeordnet, dass die elastischen Filamente 6 zwischen der ersten Seite 70 der ersten Vliesstofflage 4 und der zweiten Seite 71 der zweiten Vliesstofflage 10 positioniert sind. Die ungebundenen Bereiche bilden zugleich Freiräume 47, in denen eine Vielzahl des partikelförmigen Materials 26 aufgenommen ist. Figur 3 veranschaulicht das beschriebene Punktbindemuster in einer vergrößerten Teilansicht eines Querschnittes entlang einer Linie B-B der Figur 5.

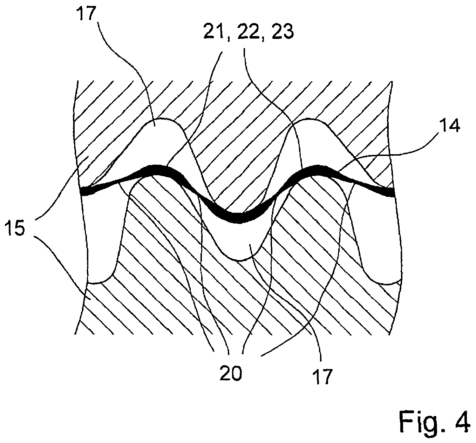

Im weiteren Verlauf wird der so gebildete Materialverbund 14 in den Spalt eines Dehnwalzenpaares 15 geführt. Figur 4 zeigt in einer vergrößerten Darstellung den Eingriffsbereich des Dehnwalzenpaares 15. Die Walzen dieses Dehnwalzenpaares 15 weisen eine gerillte Oberfläche 16 auf, wobei die Rillen 17 quer zur Längsrichtung L verlaufen. Die Rillengeometrie der Dehnwalzen ist derart aufeinander abgestimmt, dass diese berührungslos ineinander greifen können, wobei das Ausmaß des Ineinandergreifens und damit das Ausmaß das Streckens des Materialverbundes 14 regelbar ist. Die Geometrie der Rillen kann wie in Figur 4 dargestellt wellenförmig oder auch eckig gezähnt sein oder andere denkbare Konfigurationen annehmen. Wie Figur 4 veranschaulicht erfolgt das Strecken des Materialverbundes 14 derart, dass erste Bereiche 20 zwischen den Auflageabschnitten 22 des Materialsverbundes 14 auf den Rillenbergen 23 stärker gedehnt werden als zweite Bereiche 21, die mit den Auflageabschnitten 22 korrespondieren. Die Streckung des Materialverbundes 14 resultiert in einer Überdehnung der Vliesstofflagen 4, 10, da die Vliesstofflagen 4, 10 in Längsrichtung im Wesentlichen unelastisch sind. Dies bedeutet, dass die Vliesstofflagen 4, 10 eine permanente, nicht elastische Verformung erfahren. Die elastischen Filamente hingegen werden beim Strecken des Materialverbundes 14 lediglich elastisch verformt. Somit lässt sich das Vliesstoffverbundmaterial 24 nach dem Streckprozess in Längsrichtung L elastisch verformen, wobei das Ausmaß der verfügbaren Dehnung in etwa der vorangegangenen Streckung entspricht.

Zur weiteren Veranschaulichung zeigt Figur 5 in der Aufsicht einen Teilabschnitt 30 des Materialverbundes 14 unmittelbar vor dem Hindurchführen durch den Spalt des Dehnwalzenpaares 15 mit der Ausgangslänge A = 100 cm und der Breite B = 60 cm. Die elastischen Filamente 6 weisen eine Faserstärke von 100 dtex auf. Die Fasern beider Vliesstofflagen weisen eine Faserstärke von 4 dtex auf. Im schematisch illustrierten Querschnitt entlang B-B (Figur 6) sind die beiden Vliesstofflagen 4, 10 sowie die sandwichartig dazwischen im Wesentlichen spannungslos angeordneten elastischen Filamente 6 erkennbar. Auf eine Darstellung des Punktbindemusters sowie der partikelförmigen Materialien wurde in Figur 6 verzichtet. Das Punktbindemuster im Detail zeigt Figur 3.

Figur 7 zeigt diesen Teilabschnitt 30 unmittelbar nach dem Hindurchführen durch den Spalt des Dehnwalzenpaares 15, wodurch zunächst eine Streckung des Teilabschnittes um 100 %, also auf eine Länge von 200 cm erfolgt. Hierbei sind erste Bereiche 20 der Vliesstofflage 4, 10 stärker überdehnt worden als zweite Bereiche 21 der Vliesstofflagen 4, 10. Erste und zweite Bereiche 20, 21 sind alternierend streifenförmig quer zu der Längsrichtung L angeordnet, wie in der weiter unten beschriebenen Figur 8 zu erkennen ist. Die Rückstellkräfte der mit den Vliesstofflagen 4, 10 verbundenen elastisch verformten elastischen Filamente 6 führen dazu, dass der Teilabschnitt 30 des Vliesverbundmaterials 24 sich wieder bis zur Ausgangslänge A = 100 cm zusammenzieht. Da die Streckung der Vliesstofflagen 4, 10 um 100% eine permanente Dehnung (Überdehnung) der Vliesstofflagen um ebenfalls 100% zur Folge hat, führt das gleichsam überschüssige Vliesstofflagenmaterial wie in Figur 7 erkennbar zur Fältelung/Raffung des Vliesstofflagen 4, 10. Der Abstand AB zwischen den im Wesentlichen parallel zueinander angeordneten elastischen Filamenten beträgt in Querrichtung Q 3mm. Die elastischen Filamente 6 weisen somit 0 Schnittpunkte miteinander pro cm$^2$ auf. Das Flächengewicht der Vliesstofflagen 4, 10 beträgt jeweils 18 g/m$^2$. Der Gewichtsanteil der elastischen Filamente 6 am Gesamtgewicht des Vliesstoffverbundmaterials 24 beträgt 4,5%.

Der Teilabschnitt 30 des fertigen Vliesverbundmaterials 24 lässt sich nun in Längsrichtung L auf eine Länge von 200 cm, mithin um 100% elastisch dehnen (Figur 8). Somit ist mit dem vorliegenden Verfahren im Vergleich zum bekannten "Stretch-Bonding" eine wesentliche Materialeinsparung von bis zu 100% möglich, da beim "Stretch-Bonding" ein auf 200 cm ausdehnbarer Teilabschnitt die Verwendung ebenfalls 200 cm langer Vlieslagenabschnitte erfordern würde, während das hier beschriebene Verfahren die Verwendung lediglich 100 cm langer Vlieslagenabschnitte erfordert.

Nach dem Hindurchführen durch den Spalt des Dehnwalzenpaares 15 kann es vorteilhaft sein, einer Einschnürung der Vliesverbundmaterialbahn 14 in Querrichtung Q entgegenzuwirken. Hierzu kann ein weiteres Walzenpaar 31 dienen, welches die Bahn zumindest an dessen beiden Längsrändern fixiert. Vorteilhaft ist, der Einschnürung zumindest insoweit entgegenzuwirken, dass die Einschnürung, also die Verkürzung der Bahn in Querrichtung Q, also der Breite der Bahn, bezogen auf die Ausgangsbreite maximal 50%, insbesondere weniger als 30%, weiter insbesondere weniger als 20% und ganz besonders weniger als 10% beträgt.

Schließlich wird das Vliesverbundmaterial 24 im dargestellten Fall (Figur 1) auf eine Rolle 32 mit der Bahngeschwindigkeit v2 endlos aufgewickelt. Die Geschwindigkeit v2 wird hierbei vorzugsweise höher gewählt als die Geschwindigkeit v1, um die Bahn mit einer gewissen Vorspannung aufwickeln zu können. Denkbar und vorteilhaft wäre auch, die Bahn des Vliesverbundmaterials 24 direkt, also ohne vorheriges Aufwickeln auf die Rolle 32, der bestimmungsgemäßen Weiterverarbeitung, beispielsweise einer schnelllaufenden Maschine zur Herstellung von Hygiene- oder OP-Bekleidungsartikeln, insbesondere pantförmigen absorbierenden Hygieneartikeln zuzuführen. Auch solchenfalls würde dies mit einer Bahngeschwindigkeit erfolgen, die höher ist als die Geschwindigkeit v1.

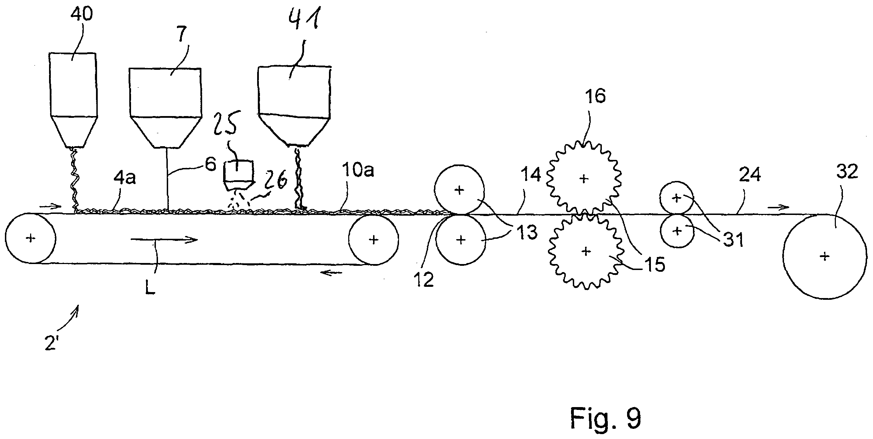

Nach einer weiteren Ausführungsform des erfindungsgemäßen Verfahrens ist denkbar und vorteilhaft, dass das Verfahren der Herstellung des Vliesstoffverbundmaterials 24 die inline-Fertigung mindestens einer, vorzugsweise beider Vliesstofflagen 4a, 10a, insbesondere nach dem Spinnvliesherstellverfahren (Spunbonding) umfasst. Die Technologie des Spunbonding ist dem Fachmann an sich geläufig. Figur 9 zeigt schematisch eine bevorzugte Anordnung der Herstellvorrichtung 2'. Anstelle der in Figur 1 dargestellten Rollen 3, 11 zum Abwickeln vorgefertigter Vliesstofflagen 4, 10 sind schematisch zwei Spinnbalken 40, 41 zum Schmelzspinnen von noch im Wesentlichen ungebundenen Spinnvlieslagen 4a und 10a dargestellt. Die Spinnvlieslagen 4a und 10a werden vorzugsweise - gleichsam in einem Arbeitsgang - durch das Hindurchführen durch das Kalanderwalzenpaar 13 vollständig gebunden und gleichzeitig miteinander und mit den elastischen Filamenten 6 verbunden. Mit gleichen Bezugszeichen versehene Vorrichtungsmodule und Materialkomponenten entsprechen ansonsten den in Figur 1 dargestellten und näher beschriebenen Vorrichtungsmodulen und Materialkomponenten.

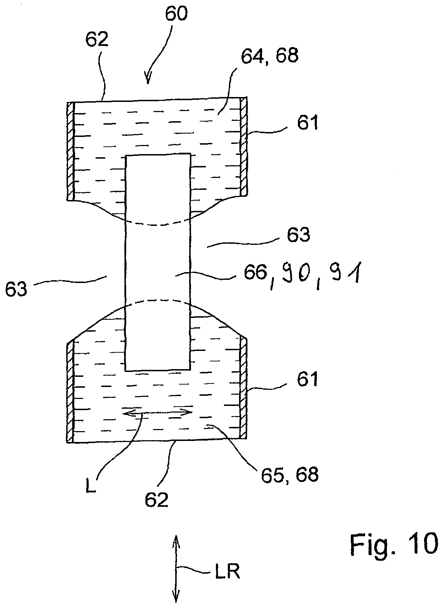

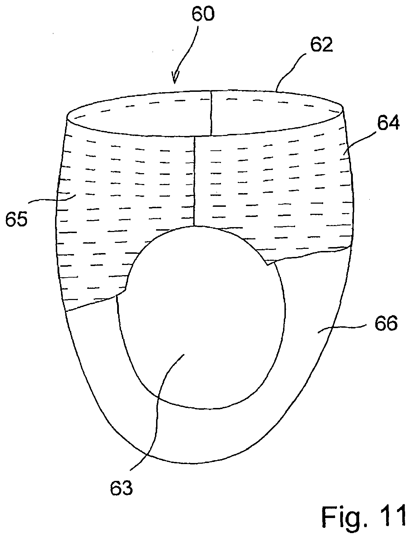

Schließlich zeigt Figur 10 einen wegwerfbaren absorbierenden Hygieneartikel 60 in Pantform jedoch im flachgelegten, ausgestreckten Zustand vor der herstellerseitigen Verbindung der Längsseitenrandabschnitte 61, mit einem eine Hüftöffnung bildenden in Umfangsrichtung nach der herstellerseitigen Verbindung der Längsseitenrandabschnitte 61 durchgehend geschlossenen Hüftrand 62 und mit Beinöffnungen 63, und mit Längsseitenrandabschnitte 61 aufweisendem Vorderteil 64 und Rückenteil 65. Vorderteil 64 und Rückenteil 65 sind voneinander in Längsrichtung LR des Hygieneartikels 60 beabstandet und werden in Längsrichtung LR des Hygieneartikels durch einen Absorptionsteil 66, der den Abstand zwischen Vorderteil 64 und Rückenteil 65 überbrückt verbunden. Figur 11 zeigt den wegwerfbaren absorbierenden Hygieneartikel 60 nach der herstellerseitigen Verbindung der Längsseitenrandabschnitte 61.

Das Absorptionsteil 66 ist auf der dem Körper zugewandten Seite des Vorder- und Rückenteils fixiert.

Das Absorptionsteil 66 kann wie dargestellt aus einem flüssigkeitsaufnehmenden und -speichernden Absorptionskern 90 bestehen oder es weist seinerseits ein flüssigkeitsundurchlässiges Backsheet und/oder ein flüssigkeitsdurchlässiges Topsheet und einen dazwischen angeordneten flüssigkeitsaufnehmenden und - speichernden Absorptionskern auf. Der Absorptionskern ist jedenfalls gebildet aus dem erfindungsgemäßen Vliesstoffverbundmaterial, wobei aus einer Vliesstoffverbundmaterialbahn 24 ein Vliesstoffverbundmaterialabschnitt 91 herausgetrennt, zum Beispiel herausgeschnitten oder herausgestanzt wurde, wobei die Längsrichtung L der Vliesstoffverbundmaterialbahn 24 im Wesentlichen senkrecht zu einer Längsrichtung LR des Hygieneartikels 60 verläuft. Die Dehnbarkeit des Absorptionskerns in Umfangsrichtung der Hüftöffnung, also senkrecht zu einer Längsrichtung LR des Hygieneartikels 60 beträgt 120%, so dass die Quererstreckung des Absorptionskerns verdoppelbar und damit einer Vielzahl von Konfektionsgrößen anpassbar ist.

Vorderteil 64 und Rückenteil 65 bestehen vorzugsweise aus einem Vliesstoffverbundmaterialabschnitt 68, welcher jeweils aus einer Vliesstoffverbundmaterialbahn, hergestellt nach einem in EP 2136753A2 beschriebenen Verfahren, herausgetrennt, zum Beispiel herausgeschnitten oder herausgestanzt wurde, wobei die Längsrichtung L der Vliesstoffverbundmaterialbahn im Wesentlichen senkrecht zu einer Längsrichtung LR des Hygieneartikels 60 verläuft. Die Erstreckung des Vorderteils 64 beträgt in Längsrichtung LR 20 cm, die des Rückenteils 65 beträgt in Längsrichtung LR 25 cm. Die Dehnbarkeit des Vorder- und Rückenteils in Umfangsrichtung der Hüftöffnung, also senkrecht zu einer Längsrichtung LR des Hygieneartikels 60 beträgt 100%, so dass der Umfang der Hüftöffnung in ähnlichem Maße wie der Absorptionskern verlängerbar und damit der Artikel insgesamt einer Vielzahl von Konfektionsgrößen anpassbar ist.

**Patentansprüche**

1. Verfahren zur Herstellung einer Bahn eines elastischen Vliesstoffverbundmaterials (24) umfassend die folgenden Schritte:

   Zuführen und Fördern einer ersten Vliesstofflage (4, 4a) in einer Längsrichtung (L);
   Zuführen einer Vielzahl elastischer Filamente (6) in der Längsrichtung (L);
   Zuführen einer zweiten Vliesstofflage (10, 10a);
   Zusammenführen der zweiten Vliesstofflage (10, 10a) mit der ersten Vliesstofflage (4, 4a) derart, dass die elastischen Filamente (6) sandwichartig zwischen der ersten Vliesstofflage (4, 4a) und zweiten Vliesstofflage (10, 10a) zur Bildung einer Lagenanordnung angeordnet werden,

   Hindurchführen der Lagenanordnung durch einen Pressspalt eines Kalanderwalzenpaares (13), zur Verbindung der Lagenanordnung zu einem Materialverbund (14) mittels eines Bindemusters, welches gebundene Bereiche (48) und ungebundene Bereichen (49) umfasst, wobei durch das Bindemuster erste Schmelzbindungen (50) direkt zwischen erster und zweiter Vliesstofflage (4, 4a, 10, 10a) erzeugt werden, und wobei die ungebundenen Bereiche (49) zwischen der ersten und der zweiten Vliesstofflage einen Freiraum (47) bilden,
   zumindest bereichsweises Überdehnen der ersten Vliesstofflage (4, 4a) und der zweiten Vliesstofflage in der Längsrichtung (L) durch Hindurchführen des Materialverbunds (14) durch den Spalt eines Paars profilierter, ineinander greifender Dehnwalzen (15), wodurch der Materialverbund (14) in der Längsrichtung (L) gestreckt wird,
   wobei die elastischen Filamente (6) in dem Vliesstoffverbundmaterial derart angeordnet sind, dass sie im Wesentlichen in der Längsrichtung (L) orientiert sind, so dass sie höchstens 10 Schnittpunkte miteinander pro cm$^2$ aufweisen,
   **dadurch gekennzeichnet, dass**
   vor dem Verbinden der Vliesstofflagen (4, 4a, 10, 10a) partikelförmige Materialien (26) auf die erste oder zweite Vliesstofflage aufgebracht werden, derart, dass die partikelförmigen Materialien (26) nach dem Verbinden der Vliesstofflagen (4, 4a, 10, 10a) sandwichartig zwischen den Vliesstofflagen (4, 4a, 10, 10a) angeordnet sind, wobei mindestens 80 Gew.-% der partikelförmigen Materialien (26) eine Partikelgröße von 45-1200 $\mu$m aufweisen und wobei nach dem Verbinden der Vliesstofflagen (4, 4a, 10, 10a) eine Vielzahl der partikelförmigen Materialien (26) innerhalb des Freiraums (47) eingebracht ist.

2. Verfahren nach Anspruch 1, **dadurch gekennzeichnet, dass** das Vliesstoffverbundmaterial (24) in der Längsrichtung (L) eine Dehnbarkeit von 35-300%, vorzugsweise von 50-200% aufweist.

3. Verfahren nach einem der Ansprüche 1 bis 2, **dadurch gekennzeichnet, dass** die partikelförmigen Materialien superabsorbierende oder geruchsadsorbierende Materialien sind.

4. Verfahren nach einem der Ansprüche 1-3, **dadurch gekennzeichnet, dass** das Verfahren das Extrudieren der elastischen Filamente (6) umfasst.

5. Verfahren nach einem der Ansprüche 1-4, **dadurch gekennzeichnet, dass** die Bahn des Vliesstoffverbundmaterials (24) nach dem Überdehnen der ersten und zweiten Vliesstofflage (4, 4a, 10, 10a) in seiner Quererstreckung fixiert wird, so dass einer Einschnürung der Bahn in Querrichtung (Q) entgegengewirkt wird.

6. Verfahren nach einem der Ansprüche 1-5, **dadurch gekennzeichnet, dass** erste Bereiche (20) der ersten und zweiten Vliesstofflage (4, 4a, 10, 10a) stärker überdehnt werden als zweite Bereiche (21) der Vliesstofflagen (4, 4a, 10, 10a).

7. Verfahren nach Anspruch 6, **dadurch gekennzeichnet, dass** die zweiten Bereiche (21) im Wesentlichen nicht überdehnt werden.

8. Verfahren nach Anspruch 6 oder 7, **dadurch gekennzeichnet, dass** die ersten Bereiche (20) und die zweiten Bereiche (21) der ersten und zweiten Vliesstofflage (4, 4a, 10, 10a) alternierend streifenförmig quer zu der Längsrichtung (L) angeordnet werden.

9. Verfahren nach einem der Ansprüche 1-8, **dadurch gekennzeichnet, dass** der Materialverbund (14) beim Hindurchführen durch den Spalt eines Paars profilierter, ineinander greifender Dehnwalzen (15) um 35-300%, insbesondere um 50-200% gestreckt wird.

10. Verfahren nach einem der Ansprüche 1-9, **dadurch gekennzeichnet, dass** die Stärke der elastischen Filamente (6) 4-700 dtex, insbesondere 50-300 dtex, weiter insbesondere 100-200 dtex beträgt.

11. Verfahren nach einem der Ansprüche 1-10 , **dadurch gekennzeichnet, dass** die elastischen Filamente (6) ein thermoplastisches, insbesondere extrudierbares Polymer ausgewählt aus der Gruppe Polyurethane, elastische Polyester, elastische Polyamide, elastische Polyolefine, elastische Blockcopolymere, enthalten oder daraus bestehen.

12. Elastisches Vliesstoffverbundmaterial (24) herstellbar oder hergestellt nach einem Verfahren gemäß einem oder mehreren der Ansprüche 1-11, wobei das Vliesstoffverbundmaterial eine erste Vliesstofflage (4, 4a), eine zweite Vliesstofflage (10, 10a), einer Vielzahl elastischer Filamente (6) sowie partikelförmige Materialien (26) umfasst, wobei zumindest 80 Gew.-% der partikelförmigen Materialien (26) eine Partikelgröße von 45-1200 μm aufweisen, wobei das Vliesstoffverbundmaterial (24) mittels eines Bindemusters, welches gebundene Bereiche (48) und diese umgebende ungebundene Bereichen (49) umfasst, verbunden ist, wobei durch das Bindemuster erste Schmelzbindungen (50) direkt zwischen erster und zweiter Vliesstofflage (4, 4a, 10, 10a) gebildet sind,

und wobei die ungebundenen Bereiche (49) zwischen der ersten und der zweiten Vliesstofflage einen Freiraum (47) bilden,

und wobei eine Vielzahl der partikelförmigen Materialien (26) innerhalb dieses Freiraums (47) angeordnet ist, wobei die elastischen Filamente (6) in dem Vliesstoffverbundmaterial derart angeordnet sind, dass sie im Wesentlichen in der Längsrichtung (L) orientiert sind, so dass sie höchstens 10 Schnittpunkte miteinander pro cm² aufweisen und wobei die erste und zweite Vliesstofflage durch den Spalt eines Paars profilierter, ineinander greifender Dehnwalzen (15) hindurchgeführt und dadurch überdehnt sind.

13. Vliesstoffverbundmaterial (24) nach Anspruch 12, **dadurch gekennzeichnet, dass** die elastischen Filamente (6) in Querrichtung einen Abstand von 0,5-10 mm, vorzugsweise von 1-5 mm, vorzugsweise von 1,2-3,5 mm voneinander aufweisen.

14. Vliesstoffverbundmaterial (24) nach einem der vorstehenden Ansprüche 12 oder 13, **dadurch gekennzeichnet, dass** der Anteil des Gewichts der elastischen Filamente (6) am Gesamtgewicht des Vliesstoffverbundmaterials (24) weniger als 40 %, vorzugsweise weniger als 30 %, weiter vorzugsweise weniger als 20 %, weiter vorzugsweise weniger 15 %, weiter vorzugsweise weniger als 10 %, insbesondere aber mehr als 3 % beträgt.

15. Vliesstoffverbundmaterial (24) nach einem der vorstehenden Ansprüche 12-14, **dadurch gekennzeichnet, dass** die erste Vliesstofflage (4, 4a) und vorzugsweise auch die zweite Vliesstofflage (10, 10a) Fasern mit einem ersten Titer, gemessen in dtex, enthalten oder daraus bestehen und die elastischen Filamente (6) einen zweiten Titer aufweisen, wobei der zweite Titer größer ist, vorzugsweise um bis zu 100% größer, weiter vorzugsweise um bis zu 200% größer, weiter vorzugsweise um bis zu 500% größer, insbesondere jedoch um höchstens 5000%, vorzugsweise um höchstens 4000 %, weiter vorzugsweise um höchstens 3000% größer ist als der erste Titer.

16. Vliesstoffverbundmaterial (24) nach einem der vorstehenden Ansprüche 12-15, **dadurch gekennzeichnet, dass** die partikelförmigen Materialien superabsorbierende Materialien umfassen oder daraus bestehen.

**Claims**

1. Process for producing a web of an elastic nonwoven composite material (24), comprising the following steps:

supplying and conveying a first nonwoven ply (4, 4a) in a longitudinal direction (L);

supplying a multitude of elastic filaments (6) in the longitudinal direction (L);

supplying a second nonwoven ply (10, 10a);

combining the second nonwoven ply (10, 10a) with the first nonwoven ply (4, 4a) in such a way that the elastic filaments (6) are arranged in a sandwich-like manner between the first nonwoven ply (4, 4a) and second nonwoven ply (10, 10a) to form a layer arrangement,

passing the layer arrangement through a press nip of a calender roll pair (13) for bonding of the layer arrangement to form a material composite (14) by means of a binding pattern comprising bonded regions (48) and unbonded regions (49), where the binding pattern produces first melt bonds (50) directly between the first and second nonwoven plies (4, 4a, 10, 10a), and where the unbonded regions (49) form a clear space (47) between the first and second nonwoven plies,

overstretching at least regions of the first nonwoven ply (4, 4a) and the second nonwoven ply in the longitudinal direction (L) by passing the material composite (14) through the nip of a pair of profiled, intermeshing stretching rolls (15), which extends the material composite (14) in the longitudinal direction (L),

where the elastic filaments (6) are arranged in the nonwoven composite material such that they are oriented essentially in the longitudinal direction (L), such that they have at most 10 points of intersection with one another per cm$^2$, **characterized in that**

prior to the bonding of the nonwoven plies (4, 4a, 10, 10a) particulate materials (26) are applied to the first or second nonwoven ply in such a way that the particulate materials (26) after the bonding of the nonwoven plies (4, 4a, 10, 10a) are arranged in a sandwich-like manner between the nonwoven plies (4, 4a, 10, 10a), where at least 80% by weight of the particulate materials (26) has a particle size of 45-1200 μm and where, after the bonding of the nonwoven plies (4, 4a, 10, 10a), a multitude of the particulate materials (26) has been introduced within the clear space (47).

2. Process according to Claim 1, **characterized in that** the nonwoven composite material (24) has an extensibility in the longitudinal direction (L) of 35-300%, preferably 50-200%.

3. Process according to either of Claims 1 and 2, **characterized in that** the particulate materials are superabsorbent or odour-absorbing materials.

4. Process according to any of Claims 1-3, **characterized in that** the process comprises the extruding of the elastic filaments (6).

5. Process according to any of Claims 1-4, **characterized in that** the web of the nonwoven composite material (24), after the overstretching of the first and second nonwoven plies (4, 4a, 10, 10a), is fixed in its transverse extent, so as to counteract constriction of the web in transverse direction (Q).

6. Process according to any of Claims 1-5, **characterized in that** first regions (20) of the first and second nonwoven plies (4, 4a, 10, 10a) are more significantly overstretched than second regions (21) of the nonwoven plies (4, 4a, 10, 10a).

7. Process according to Claim 6, **characterized in that** the second regions (21) are essentially not overstretched.

8. Process according to Claim 6 or 7, **characterized in that** the first regions (20) and the second regions (21) of the first and second nonwoven plies (4, 4a, 10, 10a) are arranged in the form of alternating stripes transverse to the longitudinal direction (L).

9. Process according to any of Claims 1-8, **characterized in that** the material composite (14), when passing through the nip of a pair of profiled, intermeshing stretching rolls (15), is stretched by 35-300%, especially by 50-200%.

10. Process according to any of Claims 1-9, **characterized in that** the thickness of the elastic filaments (6) is 4-700 dtex, especially 50-300 dtex, more especially 100-200 dtex.

11. Process according to any of Claims 1-10, **characterized in that** the elastic filaments (6) comprise or consist of a thermoplastic, especially extrudable, polymer selected from the group of polyurethanes, elastic polyesters, elastic polyamides, elastic polyolefins, elastic block copolymers.

12. Elastic nonwoven composite material (24) producible or produced by a process according to one or more of Claims 1-11, wherein the nonwoven composite material comprises a first nonwoven ply (4, 4a), a second nonwoven ply (10, 10a), a multitude of elastic filaments (6) and particulate materials (26), wherein at least 80% by weight of the particulate materials (26) has a particle size of 45-1200 μm, wherein the nonwoven composite material (24) has been bonded by means of a binding pattern comprising bonded regions (48) surrounded by unbonded regions (49), wherein the binding pattern forms first melt bonds (50) directly between the first and second nonwoven plies (4, 4a, 10, 10a),
and wherein the unbonded regions (49) form a clear space (47) between the first and second nonwoven plies,
and wherein a multitude of the particulate materials (26) has been arranged within this clear space (47), wherein the elastic filaments (6) have been arranged within the nonwoven composite material such that they are oriented essentially in the longitudinal direction (L), such that they have at most 10 points of intersection with one another per cm$^2$, and wherein the first and second nonwoven plies are conducted through the nip of a pair of profiled, intermeshing stretching rolls (15) and are overstretched as a result.

13. Nonwoven composite material (24) according to Claim 12, **characterized in that** the elastic filaments (6) have a separation in transverse direction of 0.5-10 mm, preferably of 1-5 mm, preferably of 1.2-3.5 mm.

14. Nonwoven composite material (24) according to either of the preceding Claims 12 and 13, **characterized in that** the proportion by weight of the elastic filaments (6) in the total weight of the nonwoven composite material (24) is less than 40%, preferably less than 30%, further preferably less than 20%, further preferably less than 15%, further preferably less than 10%, but especially more than 3%.

15. Nonwoven composite material (24) according to any of the preceding Claims 12-14, **characterized in that** the first nonwoven ply (4, 4a) and preferably the second nonwoven ply (10, 10a) as well comprise or consist of fibres having a first linear density, measured in dtex, and the elastic filaments (6) have a second linear density, where the second linear density is greater, preferably up to 100% greater, more preferably up to 200% greater, more preferably up to 500% greater, but preferably at most 5000%, preferably at most 4000% and more preferably at most 3000% greater, than the first linear density.

16. Nonwoven composite material (24) according to any of the preceding Claims 12-15, **characterized in that** the particulate materials comprise or consist of superabsorbent materials.


**Revendications**

1. Procédé de fabrication d'une bande d'un matériau composite non-tissé élastique (24), comprenant les étapes suivantes :

l'introduction et le transport d'une première couche de non-tissé (4, 4a) dans une direction longitudinale (L) ;
l'introduction d'une pluralité de filaments élastiques (6) dans la direction longitudinale (L) ;
l'introduction d'une deuxième couche de non-tissé (10, 10a) ;
la réunion de la deuxième couche de non tissé (10, 10a) avec la première couche de non-tissé (4, 4a) de sorte que les filaments élastiques (6) soient agencés en sandwich entre la première couche de non-tissé (4, 4a) et la deuxième couche de non-tissé (10, 10a) pour former un agencement de couches,
le passage de l'agencement de couches dans une fente de pressage d'une paire de cylindres de calandrage (13), en vue de la liaison de l'agencement de couches en un matériau composite (14) au moyen d'un motif de liaison qui comprend des zones reliées (48) et des zones non reliées (49), des premières liaisons en masse fondue (50) étant produites directement entre la première et la deuxième couche de non-tissé (4, 4a, 10, 10a) par le motif de liaison, et les zones non reliées (49) formant un espace libre (47) entre la première et la deuxième couche de non-tissé,
la distension au moins en zones de la première couche de non-tissé (4, 4a) et de la deuxième couche de non-tissé dans la direction longitudinale (L) par passage du matériau composite (14) dans la fente d'une paire de cylindres d'allongement profilés engrenants (15), suite à quoi le matériau composite (14) est étiré dans la direction longitudinale (L),
les filaments élastiques (6) étant agencés dans le matériau composite non-tissé de manière à être essentiellement orientés dans la direction longitudinale (L), de sorte qu'ils présentent au plus 10 points d'intersection par cm$^2$ les uns avec les autres,
**caractérisé en ce qu'**avant la liaison des couches de non-tissé (4, 4a, 10, 10a), des matériaux particulaires (26) sont appliqués sur la première ou la deuxième couche de non-tissé, de sorte que les matériaux particulaires (26) soient agencés après la liaison des couches de non-tissé (4, 4a, 10, 10a) en sandwich entre les couches de non-tissé (4, 4a, 10, 10a), au moins 80 % en poids des matériaux particulaires (26) présentant une taille de particule de 45 à 1 200 $\mu$m et, après la liaison des couches de non-tissé (4, 4a, 10, 10a), une pluralité des matériaux particulaires (26) étant introduite dans l'espace libre (47).

2. Procédé selon la revendication 1, **caractérisé en ce que** le matériau composite non-tissé (24) présente dans la direction longitudinale (L) une extensibilité de 35 à 300 %, de préférence de 50 à 200 %.

3. Procédé selon l'une quelconque des revendications 1 à 2, **caractérisé en ce que** les matériaux particulaires sont des matériaux superabsorbants ou adsorbant les odeurs.

4. Procédé selon l'une quelconque des revendications 1 à 3, **caractérisé en ce que** le procédé comprend l'extrusion des filaments élastiques (6).

5. Procédé selon l'une quelconque des revendications 1 à 4, **caractérisé en ce que** la bande du matériau composite non-tissé (24) est fixée au niveau de son extension transversale après la distension de la première et de la deuxième couche de non-tissé (4, 4a, 10, 10a), de manière à contrer une striction de la bande dans la direction transversale (Q).

6. Procédé selon l'une quelconque des revendications 1 à 5, **caractérisé en ce que** des premières zones (20) de la première et de la deuxième couche de non-tissé (4, 4a, 10, 10a) sont plus fortement distendues que des deuxièmes zones (21) des couches de non-tissé (4, 4a, 10, 10a).

7. Procédé selon la revendication 6, **caractérisé en ce que** les deuxièmes zones (21) ne sont essentiellement pas distendues.

8. Procédé selon la revendication 6 ou 7, **caractérisé en ce que** les premières zones (20) et les deuxièmes zones (21) de la première et de la deuxième couche de non-tissé (4, 4a, 10, 10a) sont agencées en alternance en forme de bandes perpendiculairement à la direction longitudinale (L) .

9. Procédé selon l'une quelconque des revendications 1 à 8, **caractérisé en ce que** le matériau composite (14) est étiré de 35 à 300 %, notamment de 50 à 200 %, lors du passage dans la fente d'une paire de cylindres d'allongement profilés engrenants (15) .

10. Procédé selon l'une quelconque des revendications 1 à 9, **caractérisé en ce que** l'épaisseur des filaments élastiques (6) est de 4 à 700 dtex, notamment de 50 à 300 dtex, plus particulièrement de 100 à 200 dtex.

11. Procédé selon l'une quelconque des revendications 1 à 10, **caractérisé en ce que** les filaments élastiques (6) contiennent un polymère thermoplastique, notamment extrudable, choisi dans le groupe constitué par les polyuréthanes, les polyesters élastiques, les polyamides élastiques, les polyoléfines élastiques, les copolymères séquencés élastiques, ou en sont constitués.

12. Matériau composite non-tissé élastique (24), pouvant être fabriqué ou étant fabriqué par un procédé selon une ou plusieurs des revendications 1 à 11, le matériau composite non-tissé comprenant une première couche de non-tissé (4, 4a), une deuxième couche de non-tissé (10, 10a), une pluralité de filaments élastiques (6), ainsi que des matériaux particulaires (26), au moins 80 % en poids des matériaux particulaires (26) présentant une taille de particule de 45 à 1 200 $\mu$m, le matériau composite non-tissé (24) étant relié par un motif de liaison, qui comprend des zones reliées (48) et des zones non reliées (49) entourant celles-ci, des premières liaisons en masse fondue (50) étant formées directement entre la première et la deuxième couche de non-tissé (4, 4a, 10, 10a) par le motif de liaison,
et les zones non reliées (49) formant un espace libre (47) entre la première et la deuxième couche de non-tissé,
et une pluralité des matériaux particulaires (26) étant agencée dans cet espace libre (47), les filaments élastiques (6) étant agencés dans le matériau composite non-tissé de manière à être essentiellement orientés dans la direction longitudinale (L), de sorte qu'ils présentent au plus 10 points d'intersection par cm$^2$ les uns avec les autres, et la première et la deuxième couche de non-tissé étant passées dans la fente d'une paire de cylindres d'allongement profilés engrenants (15), et ainsi distendues.

13. Matériau composite non-tissé (24) selon la revendication 12, **caractérisé en ce que** les filaments élastiques (6) présentent les uns par rapport aux autres dans la direction transversale un écart de 0,5 à 10 mm, de préférence de 1 à 5 mm, de préférence de 1,2 à 3,5 mm.

14. Matériau composite non-tissé (24) selon l'une quelconque des revendications 12 ou 13 précédentes, **caractérisé en ce que** la proportion du poids des filaments élastiques (6) par rapport au poids total du matériau composite non-tissé (24) est de moins de 40 %, de préférence de moins de 30 %, de manière davantage préférée de moins de 20 %, de manière davantage préférée de moins de 15 %, de manière davantage préférée de moins de 10%, notamment toutefois de plus de 3 %.

15. Matériau composite non-tissé (24) selon l'une quelconque des revendications 12 à 14 précédentes, **caractérisé en ce que** la première couche de non-tissé (4, 4a) et de préférence également la deuxième couche de non-tissé (10, 10a) contiennent des fibres ayant un premier titre, mesuré en dtex, ou en sont constituées, et les filaments élastiques (6) présentent un deuxième titre, le deuxième titre étant plus grand, de préférence jusqu'à 100% plus grand, de manière davantage préférée jusqu'à 200 % plus grand, de manière davantage préférée jusqu'à 500 % plus grand, notamment toutefois au plus 5 000 %, de préférence au plus 4 000 %, de manière davantage préférée au plus 3000 % plus grand que le premier titre.

16. Matériau composite non-tissé (24) selon l'une quelconque des revendications 12 à 15 précédentes, **caractérisé en ce que** les matériaux particulaires comprennent des matériaux superabsorbants ou en sont constitués.

Fig. 1

Fig. 2

Fig. 3

EP 2 566 427 B1

Fig. 4

Fig. 5

Fig. 7

Fig. 8

Fig. 6

EP 2 566 427 B1

Fig. 9

Fig. 10

Fig. 11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1184017 A1
- EP 1199058 A1
- EP 1308148 A2
- WO 0188245 A
- EP 2136753 A2
- EP 1806989 A1
- WO 06124092 A